# EUROPEAN PATENT SPECIFICATION

(11) **EP 1 462 080 B1**
(45) Date of publication and mention of the grant of the patent: **11.04.2007**
(21) Application number: 04013900.8
(22) Date of filing: 26.04.2000
(51) Int. Cl.: A61K 6/083

(54) **Self-adhesive polymerizable monomer and dental/medical compositions therefrom**
Selbstklebendes polymerisierbares Monomer und damit hergestellte Dental/Medizinische Zusammensetzungen
Monomère autoadhésif polymerisable et compositions dentaires/medicales obtenues à partir du dit monomère

(30) Priority: 12.05.1999 US 133824 P; 03.04.2000 US 541831
(43) Date of publication of application: 29.09.2004
(62) Divisional of application: 00928409.2
(73) Proprietor: DENTSPLY INTERNATIONAL, INC., York, PA 17405-0872 (US)
(72) Inventor: Klee, Joachim E., 78315 Radolfzell (DE); Walz, Uwe, 7845 Konstanz (DE)
(74) Representative: Hartz, Nikolai

(56) References cited:
- EP-A- 0 088 527
- EP-A- 0 554 890
- WO-A-98/46196

## Description

### TECHNICAL FIELD

The invention relates to self-adhesive polymerizable monomers suitable for dental and medical applications.

### BACKGROUND OF THE INVENTION

Conventional dental/medical compositions such as cements are either water-based ionic cements or resin based materials. The water-based cements have the advantage of a modest adhesion to hard tooth tissues and of a high fluoride ion release from inorganic filler material. They have the disadvantage of high water solubility, low abrasion resistance and an excessive opacity. The resin-based materials have the advantage of excellent mechanical properties, a suitable opacity and low water solubility. They have the disadvantage of a lack of adhesion, a very poor release of fluoride ions from an inorganic filler and a high shrinkage.

It has already been known to improve the adhesion of resin-based dental/medical composites by incorporation of phosphoric acid ester groups or carboxylic ester groups (US 399754, US 2709690, US 4514342, US 4806381, DE-OS 3536077, DYE-OPS 2711234, EP 0058483, DE-OS 3150285, E. Masuhara et.al. Rep. Inst. Med. DenLEng. 1 (1967) 29-33, M. Takeyama et. al. J.Soc.Dent.Appar. 19 (1978) 179-185). However, the adhesion is still insufficient. Moreover compositions with such monomers have a high shrinkage during polymerization.

EP-A-0 554 890 and EP-A-0 088 527 disclose dental adhesive compositions comprising dipentaerythritol pentacrylate phosphoric acid ester and pentaerythritol trimethacrylate phosphate, both compounds lacking any flexible spacer and the specific polymerizable trimethacrylate monomer backbone of the monomers disclosed herein. WO 98/46196 A discloses multifunctional polymerizable compounds having at least three (meth)acrylate moieties and a phosphate moiety for dental adhesive compositions.

It is a problem of the invention to provide a self-adhesive polymerizabe monomer for dental compositions and a process for preparing said self-adhesive polymerizabe monomer, whereby the dental/medical composition which combine the favourable characteristics of water-based and resin-based conventional materials and have a high adhesion to hard dental tissue and lower shrinkage.

This problem is solved by macromonomers of claim 1, the process of claims 5 and the compositions of claims 8, 14, 15, 16, 17.

### DESCRIPTION OF THE INVENTION

A self-adhesive polymerizable monomer that is characterized by the following formula: 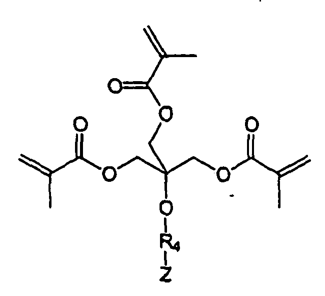 wherin
R₄ denotes a substituted or unsubstituted alkylene having 3 to 20 carbon atoms, Z is an acidic moiety selected from the group 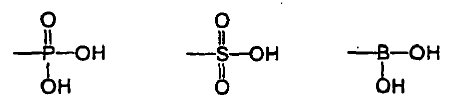 or an ionic moiety selected from the group 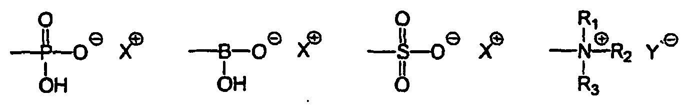
wherein
the R₁, R₂, R₃ are the same or different substituted or unsubstituted residues of Hydrogen or an alkylene having 2 to 20 carbon atoms, a cycloalkylene having 5 to 20 carbon atoms, an aryl or a heteroaryl having 4 to 12 carbon atoms,
X is a cation such as ammonium, sulfonium, sodium, potassium, strontium, calcium or magnesium salts 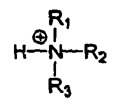 and
Y is an anion such as OH-, F-, Cl-, Br-, Tosyl-.

Most preferably the self-adhesive polymerizable monomer is characterized in the following formula:

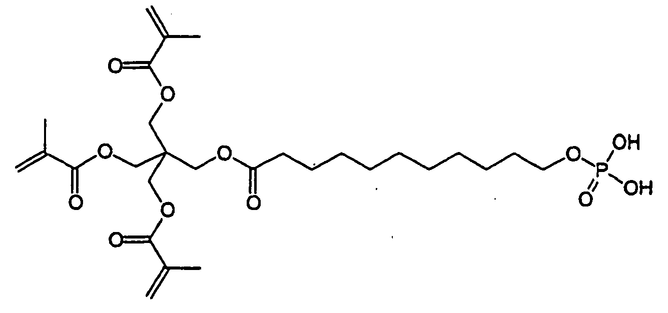

The self-adhesive polymerizable monomer is used in a dental/medical composition comprising a filler, a polymerizable monomer, a polymerization initiator and a stabilizer.

The polymerizable monomer preferably is a mono- and polyfunctional (meth)-acrylate, such as a reaction product of a polyepoxide with (meth)acrylic acid), a polyalkylenoxide di- and poly(meth)acrylate, an urethane di- and poly(meth) acrylate, preferably were used Bis-GMA diethylenglycol dimethacrylate, triethylenglycol dimethacrylate, 3,(4),8,(9)-dimethacryloyloxymethyltricyclodecane, dioxolan bismethacrylate, glycerol trimethacrylate, furfuryl methacrylate in a content of 5 to 80 wt-%.

The polymerization initiator is a thermal initiator, a redox-initiator or a photo initiator.

As filler is an inorganic filler and/or an organic filler.

The self-adhesive polymerizable monomer is usable in a self-adhesive dental/medical composition characterized by the combination of
A an adhesion to dentine of at least 5 MPa
B a fluoride release of at least 1 µg F- per week and per cm² of the exposed surface of the composition
C an opacity of at least C_{0,7} = 40 % and
D a compressive strength of at least 200 MPa.

The self-adhesive polymerizable monomer is usable in a self-adhesive dental/medical cement comprising
25 to 50 wt-% of a self-adhesive polymerizable monomer,
0 to 25 wt-% of a polymerizable monomer,
50 to 75 wt-% of a filler and polymerization initiator and stabilizers.

The self-adhesive polymerizable monomer is usable in a self-adhesive dental/medical composite comprising
15 to 50 wt-% of a self-adhesive polymerizable monomer of claims 1 to 3,
0 to 35 wt-% of a polymerizable monomer,
50 to 85 wt-% of a filler and polymerization initiator and stabilizers.

The self-adhesive polymerizable monomer is usable in a self-adhesive dental/medical sealant comprising
45 to 90 wt-% of a self-adhesive polymerizable monomer of claims 1 to 3,
0 to 45 wt-% of a polymerizable monomer,
10 to 55 wt-% of a filler and polymerization initiator and stabilizers.

The self-adhesive polymerizable monomer is usable in a self-adhesive dental/medical primer comprising
15 to 80 wt-% of a self-adhesive polymerizable monomer of claims 1 to 3,
0 to 65 wt-% of a polymerizable monomer,
0 to 65 wt-% of a filler and polymerization initiator and stabilizers
20 to 85 wt-% of a diluent and polymerization initiator and stabilizers.

An adhesive dental composite containing radioopac fillers provides a radio-opacity of at least 2 mm/ mm Al, preferably at least 3 to 7 mm/mm Al, most preferably at least 7 mm/mmAl.

The self-adhesive dental/medical composites have a fluoride release of at least 1 µg/cm², preferably at least 1-3 µg/cm², most preferably at least 3-10 µg/cm².

Furthermore the self-adhesive dental/medical composites show a opacity of at least 40 %, preferably at least 20-40 %, most preferably at least 5-20 %.

### Example 1

### Glycerine dimethacrylate-11-bromoundecanoic acid ester

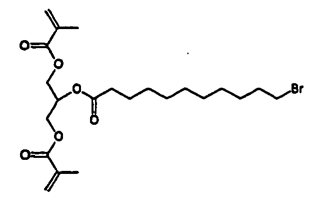

In a 1-1-three necked bottle equipped with a thermometer, condenser and stirrer 75.00 g (0.283 mol) 11-bromoundecanoic acid, 64.55 g (0.283 mol) glycerine dimethacrylate and 1.70 g dimethylamino pyridine were dissolved in 250 ml methylene chloride and cooled to 0 - 5 °C. Under stirring a solution of 64.23 g (0.311 mol) dicyclohexyl carbodiimid were added drop-wise. The resulting suspension was stirred for three hours at 20°C. Then the precipitated solid was removed by filtration and washed three times with 20 ml methylene chloride. The filtrate was cooled to 0 °C over night. The precipitated solid was removed again. Thereafter the solution was washed with 75 ml In HCl, In NaHCO₃-solution and 2 times with 150 ml water. After drying the organic solution over NaSO₄, 0.1345 g BHT were added and the solvent was removed by vacuum destillation (min. 30 mbar, at 40 °C).
Yield: 118.67 g (88.3 % of th.)

### Glycerine dimethacrylate-11-triethylammonium bromo undecanoic acid ester

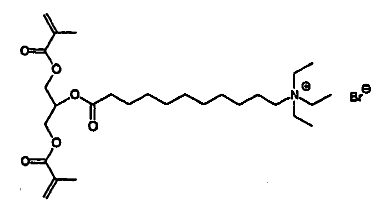

A mixture of 23.77 g (0.050 mol) glycerine dimethacrylate-11-bromoundecanoic acid ester, 6.07 g (0.060 mol) triethylamine in 50 ml ethanol was reflued for 6 hours. Then ethanol and excessive triethylamine was destilled off at 50 to 70 °C and 18 mbar.
Yield: 26.58 g (92.2 % of th.)
IR: 2600 - 2800 cm⁻¹ (absorption of the ammonium salt)

### Example 2

### Glycerine dimethacrylate-11-hydroxy undecanoic acid ester

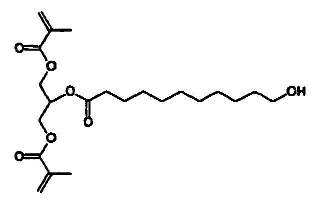

### Preparation of Glycerine dimethacrylate-11-bromoundecanoic acid ester according example 1.

To 15.00 g (0.032 mol) glycerine dimethacrylate-11-bromoundecanoic acid ester dissolved in 50 ml ethanol were added 25 ml of sodium hydoxide solution comprising 1.262 g (0.032 mol) NaOH. The mixture was shaked for one hour. Then the crude product was extracted with methylene chloride. The obtained solution was dried over NaSO4 and the solvent was remouved.
Yield: 12.50 g (96.0 % of th.)
IR: 3430 cm⁻¹ (OH)

### Glycerine dimethacrylate-11-(phosphoric acid) undecanoic acid ester

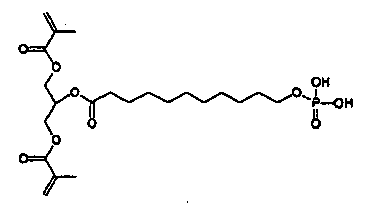

### Esterification of the hydroxyl groups of the glycerine dimethacrylate-11-hydroxy undecanoic acid ester with POCl₃.

To 11.551 g (0.028 mmol) of glycerine dimethacrylate-11-hydroxy undecanoic acid ester dissolved in 50 ml THF were added 3.117 g triethylamine in 10 ml THF. After adding 4.723 g POCl₃ (0.031 mmol) drops by drops while stirring at 0° to 5 °C the solution is stirred for further two hours at room temperature. Than the triethylamine hydrochloride is filtered off and the mixture is hydrolysed with 20 ml water. The organic solution is extracted three times with Na₂CO₃ solution and is separated from water. From the solution, dried over MgSO₄, the solvent is evaporated and the macromonomer is dried.

In the IR-spectrum the newly self-adhesive polymerizabe monomer containing phosphoric ester units shows no absorption of hydroxyl groups at □=3400 cm⁻¹. New absorptions were found at □= 1007 cm⁻¹, □= 2362 cm⁻¹ and as shoulder at □= 3302 cm⁻¹. In the ¹H NMR spectrum signals of the olefinic double bonds at □_{(CH2=)}= 6.06/6.12 ppm and at □_{(CH2=)}= 5.58/5.59 ppm were found. The signals of the methine protons (CH-OP) appears at □_{(CH)}= 5.22 and 5.88 ppm. Those of unreacted macromonomer (CH-OH) appears at □_{(CH)}= 4.34/4.35 ppm.

### Application Example 1 (Dental adhesive)

1,242 g of the obtained self-adhesive polymerizabe monomer example 2, 0,411 g triethylenglycol dimethacrylate, 0,008 g N,N-bis(C-hydroxyethyl)-p-toluidine and 0,006 g camphor quinone were homogeneously mixed. This mixture was applied in a ring (2mm high, 5 mm i.d.) on the surface of teeth and exposed with visible light (irradiation lamp Prismetics Lite De Trey Dentsply) for 40 seconds. Immediately after fixation, the teeth were transfered for 24 hours to a chamber at 37 ± 2 °C and 100 % relative humidity. The adhesion measured with a Zwick-apparatus is 5.74 ± 1.29 MPa.

### Comparative Example 1

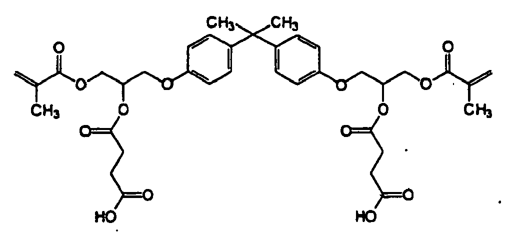

2,420 g of 2,2-Bis-[p-(2-hydroxy-3-methacryloyloxypropoxy)-phenyl]-propan (Bis-GMA) which is modified with succinic anhydride at the hydroxyl groups, 0,821 g tri-ethylenglycol dimethacrylate, 0,016 g N,N-bis(□-hydroxyethyl)-p-toluidine and 0,012 g camphor quinone were homogeneously mixed. This mixture was applied in a ring (2mm high, 5 mm i.d.) on the surface of teeth and exposed with visible light (irradia-tion lamp Prismetics Lite De Trey Dentsply) for 40 seconds. Immediately after fixa-tion, the teeth were transferred for 24 hours to a chamber at 37 ± 2 °C and 100 % relative humidity. The adhesion measured with a Zwick-apparatus is 0.45 ± 0.20 MPa.

## Claims

1. A self-adhesive polymerizable monomer represented by the following formula 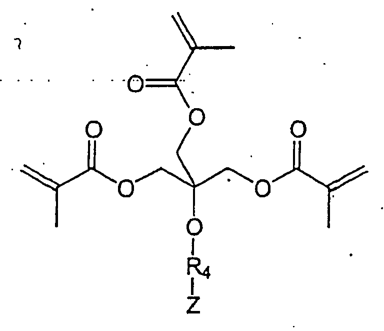 wherein
R₄ denotes a substituted or unsubstituted C₃ to C₂₀ alkylene, C₅ to C₂₀ substituted or unsubstituted cycloalkylene,
Z is an acidic moiety selected from the group consisting of 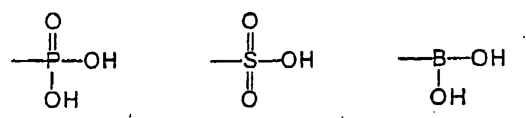 or an ionic moiety selected from the group consisting of 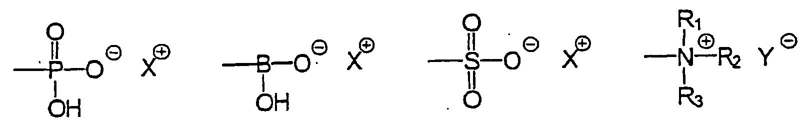
wherein
the R₁, R₂, R₃ are the same or different substituted or unsubstituted residues of hydrogen or an alkylene having 2 to 20 carbon atoms, a cycloalkylene having 5 to 20 carbon atoms, an aryl or a heteroaryl having 4 to 12 carbon atoms,
X is a cation, preferably ammonium, sulfonium, sodium, potassium, strontium, calcium or magnesium salts 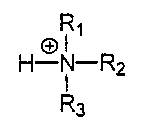 and
Y is an anion selected from the group consisting of OH-, F-, Cl-, Br, and Tosyl-.

2. A self-adhesive polymerizable monomer of claim 1 having the formula:

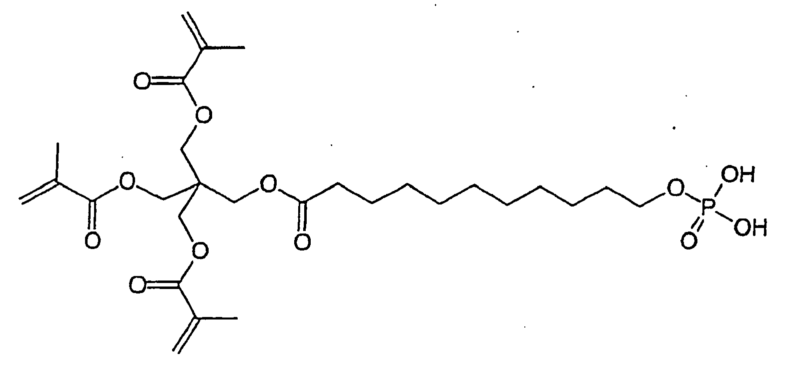

3. A self-adhesive dental/medical composition comprising a self-adhesive polymerizable monomer according to one of claim 1 or 2, a filler, a polymerizable monomer, a polymerization initiator and a stabilizer.

4. The self-adhesive dental/medical composition according to claim 3 **characterized in that** the polymerizable monomer is a mono- and polyfunctional (meth)-acrylate.

5. The self-adhesive dental/medical composition according to claim 3 **characterized in that** the polymerization initiator is a thermal initiator, a redox-initiator or a photo initiator.

6. The self-adhesive dental/medical composition according to claim 3 **characterized in that** the filler is an inorganic filler or an organic filler.

7. The self-adhesive dental/medical composition according to claim 6 comprising a fluoride releasing inorganic filler.

8. The self-adhesive dental/medical composition according to one of claim 3 comprising the combination of
A an adhesion to dentine of at least 5 MPa
B a fluoride release of at least 1 µg F⁻ per week and per cm² of the exposed surface of the composition
C an opacity of at least C_{0,7} = 40 % and
D a compressive strength of at least 200 MPa.

9. A self-adhesive dental/medical cement comprising
25 to 50 wt-% of a self-adhesive polymerizable monomer of claim 1,
0 to 25 wt-% of a polymerizable monomer,
50 to 75 wt-% of a filler and polymerization initiator and stabilizers.

10. A self-adhesive dental/medical composite comprising
15 to 50 wt-% of a self-adhesive polymerizable monomer of claim 1,
0 to 35 wt-% of a polymerizable monomer,
50 to 85 wt-% of a filler and polymerization initiator and stabilizers.

11. A self-adhesive dental/medical sealant comprising
45 to 90 wt-% of a self-adhesive polymerizable monomer of claim 1,
0 to 45 wt-% of a polymerizable monomer,
10 to 55 wt-% of a filler and polymerization initiator and stabilizers.

12. A dental/medical primer comprising
15 to 80 wt-% of a self-adhesive polymerizable monomer of claim 1,
0 to 65 wt-% of a polymerizable monomer,
0 to 65 wt-% of a filler and polymerization initiator and stabilizers
20 to 85 wt-% of a diluent and polymerization initiator and stabilizers.

## Patentansprüche

1. Selbstklebendes polymerisierbares Monomer der folgenden Formel 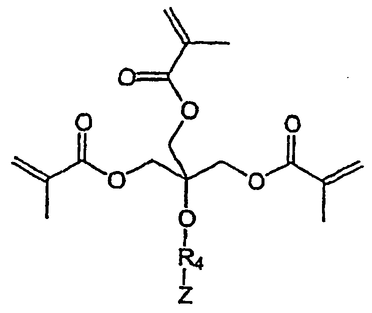 worin bedeuten
R₄ ein substituiertes oder unsubstituiertes C₃-C₂₀-Alkylen, C₅-C₂₀-substituiertes oder unsubstituiertes Cycloalkylen,
Z eine saure Struktureinheit, ausgewählt aus der Gruppe bestehend aus 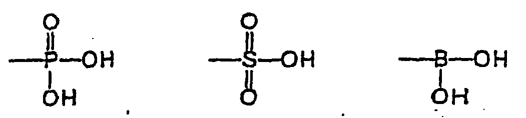 oder eine ionische Struktureinheit, ausgewählt aus der Gruppe bestehend aus 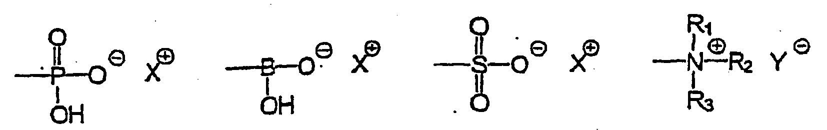
worin
R₁, R₂, R₃ gleiche oder verschiedene substituierte oder unsubstituierte Reste von Wasserstoff oder einem Alkylen mit 2 bis 20 Kohlenstoffatomen, einem Cycloalkylen mit 5 bis 20 Kohlenstoffatomen, einem Aryl oder einem Heteroaryl mit 4 bis 12 Kohlenstoffatomen sind,
X ein Kation ist, vorzugsweise Ammonium, Sulfonium, Natrium, Kalium, Strontium, Calcium oder von Magnesiumsalzen 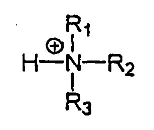
und Y ein Anion ist, ausgewählt aus der Gruppe bestehend aus OH-, F-, Cl-, Br und Tosyl-.

2. Selbstklebendes polymerisierbares Monomer nach Anspruch 1 mit der Formel:

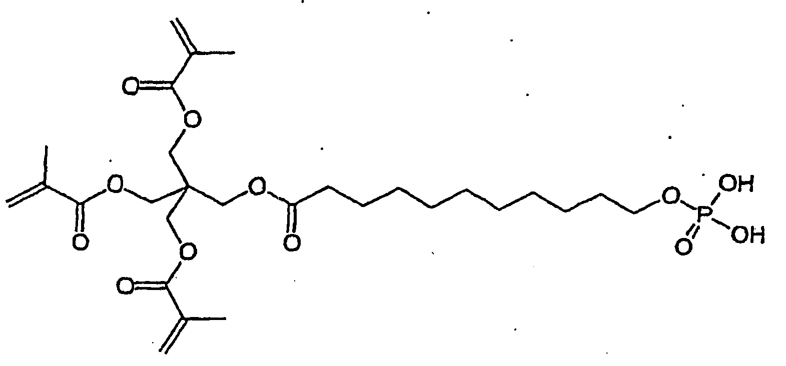

3. Selbstklebende Dental-/medizinische Zusammensetzung, umfassend ein selbstklebendes polymerisierbares Monomer nach einem der Ansprüche 1 oder 2, einen Füllstoff, ein polymerisierbares Monomer, einen Polymerisationsinitiator und einen Stabilisator.

4. Selbstklebende Dental-/medizinische Zusammensetzung nach Anspruch 3, **dadurch gekennzeichnet, dass** das polymerisierbare Monomer ein mono- und polyfunktionelles (Meth)acrylat ist.

5. Selbstklebende Dental-/medizinische Zusammensetzung nach Anspruch 3, **dadurch gekennzeichnet, dass** der Polymerisationsinitiator ein thermischer Initiator, ein Redoxinitiator oder ein Photoinitiator ist.

6. Selbstklebende Dental-/medizinische Zusammensetzung nach Anspruch 3, **dadurch gekennzeichnet, dass** der Füllstoff ein anorganischer Füllstoff oder ein organischer Füllstoff ist.

7. Selbstklebende Dental-/medizinische Zusammensetzung nach Anspruch 6, die einen Fluorid-freisetzenden anorganischen Füllstoff umfasst.

8. Selbstklebende Dental-/medizinische Zusammensetzung nach einem der Ansprüche 3 bis 7, umfassend die Kombination von
A einer Haftfestigkeit an Dentin von mindestens 5 MPa
B einer Fluorid-Freisetzung von mindestens 1 µg F⁻ pro Woche und pro cm² der exponierten Oberfläche der Zusammensetzung
C einer Opazität von mindestens C_{0,7} = 40% und
D einer Druckfestigkeit von mindestens 200 MPa.

9. Selbstklebender Dental-/medizinischer Zement, umfassend
25 bis 50 Gew.-% eines selbstklebenden polymerisierbaren Monomers des Anspruchs 1,
0 bis 25 Gew.-% eines polymerisierbaren Monomers,
50 bis 75 Gew.-% Füllstoff und Polymerisationsinitiator und Stabilisatoren.

10. Selbstklebende Dental-/medizinische Zusammensetzung, umfassend
15 bis 50 Gew.-% eines selbstklebenden polymerisierbaren Monomers nach Anspruch 1,
0 bis 35 Gew.-% eines polymerisierbaren Monomers,
50 bis 85 Gew.-% Füllstoff und Polymerisationsinitiator und Stabilisatoren.

11. Selbstklebende Dental-/medizinische Dichtungsmasse, umfassend
45 bis 90 Gew.-% eines selbstklebenden polymerisierbaren Monomers nach Anspruch 1,
0 bis 45 Gew.-% eines polymerisierbaren Monomers,
10 bis 55 Gew.-% Füllstoff und Polymerisationsinitiator und Stabilisatoren.

12. Dental-/medizinisches Grundierungsmittel, umfassend
15 bis 80 Gew.-% eines selbstklebenden polymerisierbaren Monomers nach Anspruch 1,
0 bis 65 Gew.-% eines polymerisierbaren Monomers,
0 bis 65 Gew.-% Füllstoff und Polymerisationsinitiator und Stabilisatoren,
20 bis 85 Gew.-% Verdünnungsmittel und Polymerisationsinitiator und Stabilisatoren.

## Revendications

1. Monomère polymérisable auto-adhésif représenté par la formule suivante 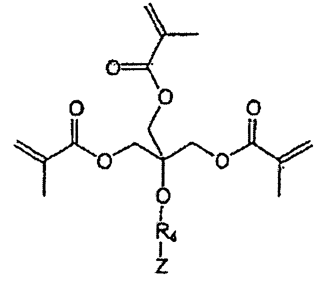 dans laquelle
R₄ représente un groupe alkylène en C₃ à C₂₀ substitué ou non substitué ou cycloalkylène en C₅ à C₂₀ substitué ou non substitué,
Z représente un groupement acide choisi dans le groupe consistant en 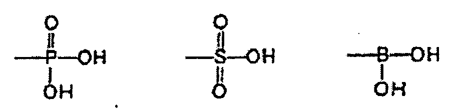
ou un groupement ionique choisi dans le groupe consistant en 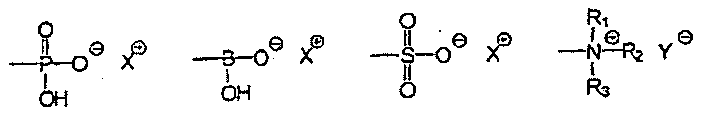
où
les groupes R₁, R₂ et R₃ représentent des résidus identiques ou différents, substitués ou non substitués, hydrogène ou alkylène ayant 2 à 20 atomes de carbone, cycloalkylène ayant 5 à 20 atomes de carbone, aryle ou hétéroaryle ayant 4 à 12 atomes de carbone,
X représente un cation, de préférence des sels d'ammonium, de sulfonium, de sodium, de potassium, de strontium, de calcium ou de magnésium 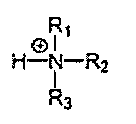 et
Y représente un anion choisi dans le groupe consistant en OH-, F-, Cl-, Br et tosyl-.

2. Monomère polymérisable autoadhésif suivant la revendication 1, de formule

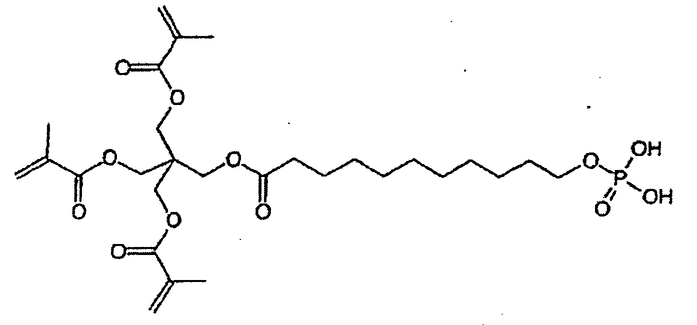

3. Composition dentaire/médicale autoadhésive comprenant un monomère polymérisable autoadhésif suivant une des revendications 1 et 2, une charge, un monomère polymérisable, un initiateur de polymérisation et un stabilisant.

4. Composition dentaire/médicale autoadhésive suivant la revendication 3, **caractérisée en ce que** le monomère polymérisable est un (méth)acrylate mono- ou polyfonctionnel.

5. Composition dentaire/médicale autoadhésive suivant la revendication 3, **caractérisée en ce que** l'initiateur de polymérisation est un initiateur thermique, un initiateur redox ou un photo-initiateur.

6. Composition dentaire/médicale autoadhésive suivant la revendication 3, **caractérisée en ce que** la charge est une charge inorganique ou une charge organique.

7. Composition dentaire/médicale autoadhésive suivant la revendication 6, comprenant une charge inorganique libérant des fluorures.

8. Composition dentaire/médicale autoadhésive suivant une de la revendication 3, comprenant l'association
A. d'une adhérence à la dentine d'au moins 5 MPa,
B. d'une libération de fluorures d'au moins 1 µg de F⁻ par semaine et par cm² de surface exposée de la composition,
C. d'une opacité d'au moins C_{0,7} = 40 % et
D. d'une résistance à la compression d'au moins 200 MPa

9. Ciment dentaire/médical autoadhésif comprenant
25 à 50 % en poids d'un monomère polymérisable autoadhésif de la revendication 1,
0 à 25 % en poids d'un monomère polymérisable,
50 à 75 % en poids d'une charge et d'un initiateur de polymérisation et de stabilisants.

10. Composite dentaire/médical autoadhésif comprenant
15 à 50 % en poids d'un monomère polymérisable autoadhésif de la revendication 1,
0 à 35 % en poids d'un monomère polymérisable,
50 à 85 % en poids d'une charge et d'un initiateur de polymérisation et de stabilisants.

11. Produit de scellement dentaire/médical autoadhésif comprenant
45 à 90 % en poids d'un monomère polymérisable autoadhésif de la revendication 1,
0 à 45 % en poids d'un monomère polymérisable,
10 à 55 % en poids d'une charge et d'un initiateur de polymérisation et de stabilisants.

12. Apprêt dentaire/médical comprenant
15 à 80 % en poids d'un monomère polymérisable autoadhésif de la revendication 1,
0 à 65 % en poids d'un monomère polymérisable,
0 à 65 % en poids d'une charge et d'un initiateur de polymérisation et de stabilisants.
20 à 85 % en poids d'un diluant et d'un initiateur de polymérisation et de stabilisants.
